# EUROPEAN PATENT APPLICATION

(11) **EP 4 140 560 A2**
(43) Date of publication of application: **01.03.2023**
(21) Application number: 20823886.5
(22) Date of filing: 23.10.2020
(51) Int. Cl.: B01D 39/08, B01D 39/16, A41D 13/11, A62B 23/02, B01D 39/18

(54) **MULTILAYER FILTER WITH ANTIMICROBIAL PROPERTIES AND USE THEREOF IN INDUSTRIAL FILTRATION APPLICATIONS AND PROTECTIVE MASKS**

(30) Priority: 20.04.2020 ES 202030319
(71) Applicant: Bioinicia, S.L., 46980 Paterna (Valencia) (ES); Consejo Superior de Investigaciones Científicas (CSIC), 28006 Madrid (ES)
(72) Inventor: LAGARÓN CABELLO, José María, 46980 Paterna (Valencia) (ES); PARDO FIGUEREZ, Maria de las Mercedes, 46980 Paterna (Valencia) (ES); CHIVA FLOR, Alberto, 46980 Paterna (Valencia) (ES)
(74) Representative: Pons
(86) International application number: PCT/ES2020/070645
(87) International publication number: WO 2021/005258

(57) **Abstract**

The present invention relates to the field of polymeric materials applied to the manufacture of materials for use in filters for filtration equipment such as respirators and for protective face masks. In particular, the invention relates to multilayer filters for respirators and protective face masks that may be biodegradable and comprise filtration materials based on ultrafine fibres obtained by electro-hydrodynamic and aerohydrodynamic processing and exert passive FFP1, FFP2, N95 and FFP3 type protection, and that may also be washable and have active antimicrobial properties.

## Description

The present invention falls within the area of polymeric materials applied to the sector of manufacturing materials for use in filters for filtration equipment such as ventilators and for protective masks. In particular, the invention relates to multilayer filters for ventilators and protective masks which can be biodegradable and which comprise filtration materials based on ultrafine fibres obtained by electrohydrodynamic and aerohydrodynamic processing and which exercise passive FFP1, FFP2, N95 and FFP3 protection and which can also be washable and have active antimicrobial properties.

### BACKGROUND OF THE INVENTION

The absorption of high-concentration airborne contaminants into the body can potentially be very dangerous and can be absorbed by the body through the skin, eyes or respiratory system. The absorption of airborne contaminant particles into the lungs through the respiratory system can lead to both acute and chronic health risks, especially when they include pathogens of respiratory infectious diseases such as tuberculosis and measles and emerging diseases such as severe acute respiratory syndrome (SARS) and H1N1 A flu.

In these cases, the size of the contaminants is important. In general, smaller particles are more likely to be airborne and more dangerous. Thus, particles larger than 10 µm generally collect at the top of the respiratory system. Therefore, most of them cannot enter deep into the lungs. However, particles smaller than 10 µm are breathable, which means that they are capable of penetrating deep into the lungs. Those particles include, among others, bacteria, viruses, clay, silt, tobacco smoke and metal fumes.

The danger of airborne contaminants can be managed by applying basic controls, such as increasing ventilation or providing workers with protective equipment such as protective masks.

Protective masks have been widely used by hospital personnel, laboratory researchers, construction site workers, as well as the general public in highly contaminated areas or during flu season.

Protective masks are generally made up of a filter barrier, which is a critical component that determines the level of protection of the mask, since filtration efficiency depends on the particle size and the speed of the air flow.

Most filter barriers of conventional protective masks are not functionalised with biocides or virucides, meaning that said protective masks are simply used as a physical barrier for filtering out contaminants, and in most cases they do not have the capacity to stop microorganisms as small as viruses, which are between 100 and 200 nm in size. Furthermore, when these contaminants are viruses and bacteria, said barriers also do not eliminate them from the fabric with which they come into contact. Therefore, the microorganisms attached to the masks can survive for several hours, greatly increasing the risk of cross infection. Finally, given that the known filters are made of non-biodegradable materials, in the case of the mass use of masks by the non-medical population, such as during a pandemic, they can end up creating a serious environmental problem.

### DESCRIPTION OF THE INVENTION

The present invention proposes a methodology to generate a multilayer filter based on ultrafine fibres and use thereof in ventilators and protective masks, which have a combination of materials, an arrangement of the same, morphology and grammage of the fibres that gives them the balance of properties necessary to achieve paraffin aerosol filtration levels, called FFP1 (with a capacity for filtering virus-containing aerosols equal to or greater than 80%), FFP2 and N95 (with a capacity for filtering virus-containing aerosols equal to or greater than 94 and 95%, respectively) and FFP3 (with a capacity for filtering virus-containing aerosols equal to or greater than 99%); and maintaining levels of maximum resistance to inhalation over areas of 55 cm², with an air flow of 30 l/min, less than 1.1 millibar, and with an air flow of 85 l/min, less than 3.5 millibars. These masks can additionally contain antimicrobial substances, be washable and can also have compostability and biodegradability properties in the environment.

Therefore, a first aspect of the present invention relates to a multilayer filter characterised in that it comprises at least:
i) An inner layer (a) characterised in that it is composed of polymeric filter materials and has a surface density of at least 0.01 g/m², more preferably between 5 and 3000 g/m², even more preferably between 20 and 300 g/m²;
ii) An intermediate layer (b) characterised in that it is composed of polymeric fibres, optionally containing antimicrobial substances, and has a surface density of at least 0.01 g/m², more preferably between 0.1 and 10 g/m², and even more preferably between 0.2 and 3 g/m²;
iii) An outer layer (c) characterised in that it is composed of polymeric filter materials and has a surface density of at least 0.01 g/m², more preferably between 5 and 3000 g/m², and even more preferably between 20 and 300 g/m².

In a preferred embodiment, the polymeric materials forming the inner layer (a) and the outer layer (c) of the filter are selected, without limitation, from non-water-soluble proteins such as keratin, polysaccharides such as celluloses, cottons and any natural fibre in general, and waxes or paraffins, polyhydroxyalkanoates (PHA) such as PHB, PHV, medium-chain-length PHA (mcl-PHA), and all the possible copolymers thereof such as PHBV, among others, poly-ε-caprolactone (PCL) and all the copolymers thereof such as PEG-PCL and PCLA, polylactic acid (PLA), all the copolymers thereof such as PGLA, polyphosphazenes, polyorthoesters, polyesters obtained from natural precursors such as polytrimethylene terephthalate (PTT), polybutylene terephthalate (PBT), polybutylene succinate (PBS), and all the possible copolymers thereof such as poly(butylene adipate-co-terephthalate) (PBAT), among others, as well as other non-biodegradable polymers, such as: polyolefins, among which it is worth noting ethylene-based polymers and copolymers, such as polyethylene, propylene, polyethylene-co-vinyl acetate (EVA), polyethylene terephthalate (PET) and copolymers thereof, silicones, polyesters, polyurethanes (PURs), polysulfones, halogenated polymers such as polyvinylidene fluoride (PVDF), polytetrafluoroethylene (PTFE) or polyvinylidene chloride (PVDC), polyvinylidene chloride (PVC), polycarbonates, acrylonitrile butadiene styrene, latex, polyimides, polysulfones, and polyamides such as PA6, PA66 or PA69, PA1010, as well as mixtures of any of the above, or any of the above mixed with additives such as plasticisers, surfactants, antioxidants, colourants, etc.

In a more preferred embodiment, the inner layer (a) and the outer layer (c) are made of woven or non-woven polymeric filter materials, with or without functional additives such as, and without limitation, hydrophobising agents, or heat- or ultrasonic-welded. In an even more preferred embodiment, they are made of non-woven polymeric filter materials.

In a more preferred embodiment, the polymeric material that makes up the inner layer (a) and the outer layer (c) are independently selected from polypropylene, polyamide, polyester, natural fibres, cotton and cellulose, or any of the combinations thereof.

In another preferred embodiment, the materials of the fibres forming the intermediate layer (b) are polymers selected from halogenated polymers such as polyvinylidene fluoride and the copolymers thereof, polytetrafluoroethylene, polyvinylidene chloride, polyacrylonitrile, polysulfones and the derivatives thereof, polylactic acid, polyurethanes and the derivatives thereof, polyamides, cross-linked polyvinyl alcohol, polyvinyl butyral, polyhydroxyalkanoates such as poly(3-hydroxybutyrate-co-3-hydroxyvalerate), polystyrene, polyvinyl acetate, polyethylene terephthalate, chitosan, polycarbonates, poly(methyl methacrylate) and polycaprolactones, or any of the combinations thereof.

In another preferred embodiment, the polymers of the fibres forming the intermediate layer (b) are selected from polyvinylidene chloride, polyacrylonitrile and polyhydroxyalkanoates, or any of the combinations thereof. In an even more preferred embodiment, the polymers that make up the intermediate layer (b) of the filter of the invention are made of polyvinylidene chloride or polyhydroxyalkanoates.

In another preferred embodiment, the polymers of the fibres forming the intermediate layer (b) are selected from halogenated polymers such as polyvinylidene fluoride and the copolymers thereof, polytetrafluoroethylene, polyvinylidene chloride, polyacrylonitrile, polysulfones and the derivatives thereof, polyurethanes and the derivatives thereof, polyamides, cross-linked polyvinyl alcohol, polyvinyl butyral, non-water-soluble proteins such as keratin, polysaccharides such as celluloses and chitosans, cottons, and waxes or paraffins, polyhydroxyalkanoates (PHA) such as PHB, PHV, medium-chain-length PHA (mcl-PHA), and all the possible copolymers thereof such as PHBV, among others, poly-ε-caprolactone (PCL) and all the copolymers thereof such as PEG-PCL and PCLA, polylactic acid (PLA), all the copolymers thereof such as PGLA, polyphosphazenes, polyorthoesters, biodegradable polyesters obtained from natural or synthetic precursors such as polytrimethylene terephthalate (PTT), polybutylene terephthalate (PBT), polybutylene succinate (PBS), polybutylene succinate adipate (PBSA) and all the possible copolymers thereof such as poly(butylene adipate-co-terephthalate) (PBAT), polystyrene, polyvinyl acetate, polyethylene terephthalate, polycarbonates, poly(methyl methacrylate) and polycaprolactones and the copolymers thereof, or any of the combinations thereof.

In another preferred embodiment, the polymers of the fibres forming the intermediate layer (b) have a molecular weight less than 800 kDalton, more preferably less than 300 kDalton, and even more preferably less than 200 kDalton.

In another preferred embodiment, the intermediate layer (b) contains particles, nanoparticles or liquids of an antimicrobial substance selected, without limitation, from zinc oxide, silver, silver nitrate, copper, copper oxide, carbonaceous materials such as graphene, carbon micro- and nanotubes, titanium oxide and dioxide, natural extracts and essential oils, chitin and chitosan, aluminium oxide, silicon dioxide (SiO₂), cyclodextrins (CD), antibiotics and antivirals such as tetracycline, iodine, triclosan, chlorhexidine, acyclovir, cyclofloxacin or combinations thereof. In a more preferred embodiment, the antimicrobial substance is zinc oxide; in an even more preferred embodiment, the antimicrobial substance contained in the intermediate layer (b) are zinc oxide nanoparticles.

In another preferred embodiment, the inner layer(s) (a) and/or outer layer(s) (c) contain particles, nanoparticles or liquids of an antimicrobial substance selected, without limitation, from zinc oxide, silver, silver nitrate, copper, copper oxide, carbonaceous materials such as graphene, carbon micro- and nanotubes, titanium oxide and dioxide, natural extracts and essential oils, chitin and chitosan, aluminium oxide, silicon dioxide (SiO₂), cyclodextrins (CD), antibiotics and antivirals such as tetracycline, iodine, triclosan, chlorhexidine, acyclovir, cyclofloxacin or combinations thereof. In a more preferred embodiment, the antimicrobial substance is zinc oxide; in an even more preferred embodiment, the antimicrobial substance contained in the inner layer(s) (a) and/or outer layer(s) (c) are zinc oxide nanoparticles.

In another preferred embodiment, the intermediate layer (b) does not contain particles, nanoparticles or liquids of an antimicrobial substance.

In another preferred embodiment, the content by weight of the antimicrobial in the fibres in each of the layers is less than 50%, more preferably less than 25% and even more preferably less than 13%.

In this invention, the term "antimicrobial substance" refers to an agent that kills microorganisms or stops their growth. Microorganisms encompass heterogeneous unicellular organisms that are evolutionarily unrelated to each other, such as bacteria (prokaryotes), protozoa (eukaryotes, some algal phylum) and single-celled fungi, and also includes ultramicroscopic acellular biological entities such as viruses and prions. The antimicrobial field of activity of this invention is mainly focused on bacteria, fungi and especially all types of viruses.

In another preferred embodiment, the fibres of the intermediate layer (b) are fibres with a smooth or beaded morphology.

In another preferred embodiment, the intermediate layer (b) comprises an additional layer. This additional layer (b') may contain the same polymer as that of the first intermediate layer (b) on which it is deposited or it may be composed of fibres of a different polymer. Likewise, the morphology of both polymers forming each of the two intermediate layers (b and b') can have the same or different morphology and, likewise, they can have the same or different surface density.

In an even more preferred embodiment, the additional intermediate layer (b') is composed of the same polymer, with the same morphology and the same surface density as the polymer in the intermediate layer (b).

In another preferred embodiment, the intermediate layer (b) has a morphology with fibres with a mean diameter of between 10 and 3000 nm, more preferably between 50 and 900 nm and even more preferably between 75 and 300 nm.

In another preferred embodiment, the fibres of the additional intermediate layer (b') have a morphology with fibres with a diameter greater than 500 nm.

In another preferred embodiment, the fibres of the additional intermediate layer (b') have a morphology with fibres with a diameter equal to that of the fibres of the intermediate layer (b).

In another preferred embodiment, when the mean fibre diameter of the intermediate layer (b) is less than 200 nm, the surface density of the intermediate layer is equal to or less than 0.5 g/m².

In another preferred embodiment of this invention, when the fibres of the polymers that make up the filter layers are smooth, the surface density of the intermediate layer is equal to or less than 1 g/m².

In another preferred embodiment of this invention, when the fibres of the polymers that make up the filter layers are beaded, the surface density of the intermediate layer is equal to or less than 3 g/m².

In the present invention, the surface density is expressed in g/m²; for each of the layers it is calculated by weighing a sample with known dimensions. This weight is then divided by the surface of the sample. This process is carried out with at least 5 samples of each layer in order to thus obtain a mean surface density value for the entire layer.

In another preferred embodiment, the dispersion of the surface density of the intermediate layer (b) is less than 30%, more preferably less than 20%, and even more preferably less than 10%.

In the present invention, the polymers that make up the filter layers are preferably compostable and/or biodegradable in the environment.

In another preferred embodiment, the multilayer filter of the present invention has levels of maximum resistance to inhalation over areas of 55 cm², with an air flow of 30 l/min, less than 1.1 millibar, and with an air flow of 85 l/min, less than 3.5 millibar.

In another preferred embodiment, the multilayer filters of the present invention can be used alone, or stacked in any possible configuration on themselves or on other commercial multilayer or monolayer filters, to form new, thicker filters with greater filtration capacity.

In this way, the filters of the present invention can be used on rolls as an intermediate product to be later optionally laminated with other layers that provide hydrophobicity, splash resistance, additional filtering capacity, mechanical strength and/or comfort upon contact with the skin, and finally cut by any industrial method in the dimensions required by the final manufacturer of the product, or they can be cut into any shape or size by any cutting method, for example, with a laser or a die cutter, with the proper dimensions and used as the final product.

In the present invention, the term "polymer" refers to macromolecular materials both in the pure ex-reactor state and as additive and post-processed materials in commercial formulas typically used by chemical industries, more commonly called plastic grades. Any of the polymers or plastic grades can be additionally added to process additives, promoters of biodegradability or those that provide stability, other type of additives of the "filler" type, either in micro-, submicro- or nanometric form to improve the physicochemical properties thereof or of retention capacity and controlled release of the antimicrobial. Such additives can be chemicals, fibres, sheets or particles.

In the present invention, the terms referring to the morphology of "smooth" and "beaded" fibres refer to different types of morphology found in the fibrous structure generated. Thus, the term "smooth fibres" refers to when the fibres have a smooth surface with a rather regular cross-section of the diameter. On the other hand, the term "beaded fibres" refers to fibres that have spherical, oblong or other irregular beads interspersed along the cross section of the fibre. By being made up of thicker beads, this structure generates additional micro-porosity that makes them advantageous to improve the breathability of the filter (ease of the fabric for air to pass through its cross section), although it reduces the resistance thereof to the penetration of aerosols, for example, paraffin or sodium chloride, (capacity of the fabric to reduce the passage of the virus through its cross section).

On the other hand, the intermediate layers of the multilayer filters of the invention can be manufactured continuously by depositing each layer on the previous one, or manufactured separately and then optionally laminating them, or by combining both.

When lamination is done by calendering, it can be done by using two or more rollers with or without pressure, wherein at least one of them can be at the required temperature, or the case may occur where all the rollers are at the same required temperature.

The produced layers can be calendered in such a way that the inner layer is in contact with the heating roller or, conversely, the last layer is the layer in contact with the roller that is at the required temperature.

In another preferred embodiment, the different layers can be manufactured continuously one on top of the previous one and then a treatment process is carried out with or without heat and with or without pressure, preferably by calendering at a low temperature, to ensure adhesion between layers, provide a smoother texture and reduce the thickness.

Regarding the manufacture of the intermediate layers that make up the mask of the invention, these layers are preferably made using any of the electrohydrodynamic and aerohydrodynamic techniques for obtaining known fibres, such as electrospinning, electrohydrodynamic direct-writing, melt electrospinning, solution blow spinning, electrospraying, solution blow spraying, electrospraying assisted by pressurised gas or combination of all the above. However, any other method for obtaining fibres may also be used, such as centrifugal jet spinning or the combination of this and those previously mentioned. Electrohydrodynamic and aerohydrodynamic techniques are based on the formation of micro-, submicro- or ultrafine polymeric fibres at room temperature or lower temperature, using a polymeric solution to which an electric field or gas pressure is applied. The fact that it is used in the form of a solution presents great versatility, since it allows various substances (antimicrobials) to be incorporated in the solution itself. At the same time, the fact that its processability is at room temperature avoids certain problems such as the degradation of active substances.

With these techniques and the aforementioned polymers, in the present invention the antimicrobial substance is incorporated by using techniques including, without limitation, core-shell technology, co-deposition, direct mixing, emulsion techniques, pre-encapsulation in particles or layer-by-layer deposition, etc.

In the present invention, the layer-by-layer deposition method consists of the use of a system in which the layers are deposited sequentially within the same process. In this way, one of the layers is initially electrospun until the desired thickness is reached and then the second layer is electrospun on top of the first layer, continuously obtaining a multilayer system *in situ.*

A second aspect of the invention relates to obtaining the multilayer filters of the invention as defined above, which comprises the following steps:
i) Depositing the intermediate layer (b) on the inner layer (a);
ii) Optionally, depositing one or more additional intermediate layers (b') on the intermediate layer (b);
iii) Laminating the outer layer (c) with the previous layers.

A third aspect of the invention relates to obtaining the multilayer filters of the invention as defined above, which comprises the following steps:
i) Depositing the intermediate layer (b) on the inner layer (a);
ii) Depositing an additional intermediate layer (b') on the inner face of the outer layer (c);
iii) Laminating the previous layers so that layers (b) and (b') are in contact.

In a preferred embodiment of the invention, the different layers that make up the filter are laminated by simply joining the layers together, without any type of adhesion method.

Optionally, the layers that make up the filter can be partially or totally laminated along the surface thereof, thus adding protective layers below the inner layer (a) and/or above the outer layer (c) by means of any known lamination technique, including calendering with or without pressure under ambient or hot conditions, by applying adhesives, with melting points, for example by ultrasound, stitching or heat-sealing.

A third aspect of the invention relates to the use of the multilayer filter of the present invention to manufacture, without limitation, generic industrial air or liquid filters, medical ventilators for patients who need artificial respiration, and to manufacture washable or non-washable masks, either for surgical, hygienic or personal protective equipment (PPE) protection. The filters of the present invention can be used on rolls as an intermediate product to be later optionally laminated with other layers that provide hydrophobicity, splash resistance, additional filtering capacity, mechanical strength and/or comfort upon contact with the skin, and finally cut by any industrial method in the dimensions required by the final manufacturer of the product, or they can be cut in any shape or size, by any cutting method, for example, with a laser or a die-cutter, with the proper dimensions and used as the final product. Therefore, they are used, without limitation, to manufacture masks made of one or more pieces according to any known industrial method, or as an expendable and therefore disposable filter for reusable masks.

In a preferred embodiment, the multilayer filters of the present invention have resistance to the penetration of virus-sized particles of less than 20%, more preferably less than 6%, and even more preferably less than 1%.

In a preferred embodiment, the multilayer filters of the present invention have levels of maximum resistance to inhalation over areas of 55 cm², with an air flow of 30 l/min, less than 1.1 millibar, and with an air flow of 85 l/min, less than 3.5 millibars.

The primary function of the multilayer filters of the invention is to therefore protect against the penetration of microorganisms, typically against viruses and bacteria (both Gram-positive and Gram-negative), although preferably against viruses sized between 30-500 nm, such as, without limitation, adenovirus, coronavirus, human metapneumovirus, parainfluenza virus, the flu (influenza), respiratory syncytial virus (RSV), rhinovirus/enterovirus and more particularly, Ebola virus, herpes virus (HSV-1), influenza virus (A, B, C, D), human respiratory syncytial virus (RSV), chickenpox, SARS-CoV and the derivatives thereof, as well as against SARS-CoV-2 which causes COVID-19.

An additional aspect of the present invention relates to a mask containing the filter of the invention as described above, wherein the mask protects against microorganisms.

In a preferred embodiment, the microorganism is a virus selected from Ebola virus, herpes virus, influenza virus, human respiratory syncytial virus, chickenpox, SARS-CoV and the derivatives thereof, as well as SARS-CoV-2 which causes COVID-19.

A final aspect of the present invention relates to generic industrial air or liquid filtration equipment that contains the filter of the invention as described above, wherein the mask protects against microorganisms.

In a preferred embodiment, the microorganism is a virus selected from Ebola virus, herpes virus, influenza virus, human respiratory syncytial virus, chickenpox, SARS-CoV and the derivatives thereof, as well as SARS-CoV-2 which causes COVID-19.

Throughout the description and in the claims, the word "comprises" and its variants are not intended to exclude other technical features, additives, components or steps. For those skilled in the art, other objects, advantages and features of the invention may be deduced from both the description and the practical use of the invention. The following examples and drawings are provided by way of illustration, and are not meant to limit the present invention.

### BRIEF DESCRIPTION OF THE FIGURES

**Figure 1** shows a diagram of the tri-layer structure with smooth ultrafine PVDF fibres.
**Figure 2** shows a diagram of the tri-layer structure with beaded ultrafine PVDF fibres.
**Figure 3** shows a diagram of the tri-layer structure with smooth ultrafine PHBV fibres.
**Figure 4** shows a diagram of the multilayer structure of sequentially electrospun PVDF and as a symmetrical sandwich.
**Figure 5** shows a diagram of the co-deposition of electrospun ultrafine PVDF and PAN fibres *in situ.*
**Figure 6** shows a diagram of the tri-layer structure with smooth PHBV microfibres.

### EXAMPLES

Next, the invention will be illustrated by some examples carried out by the inventors for each type of filter developed (e.g., passive FFP3 filter, with antimicrobial capacity and biodegradable design) which demonstrates the effectiveness of the product of the invention.

### Example 1: FPP3 tri-layer structure system with electrospun PVDF with smooth ultrafine fibre structure

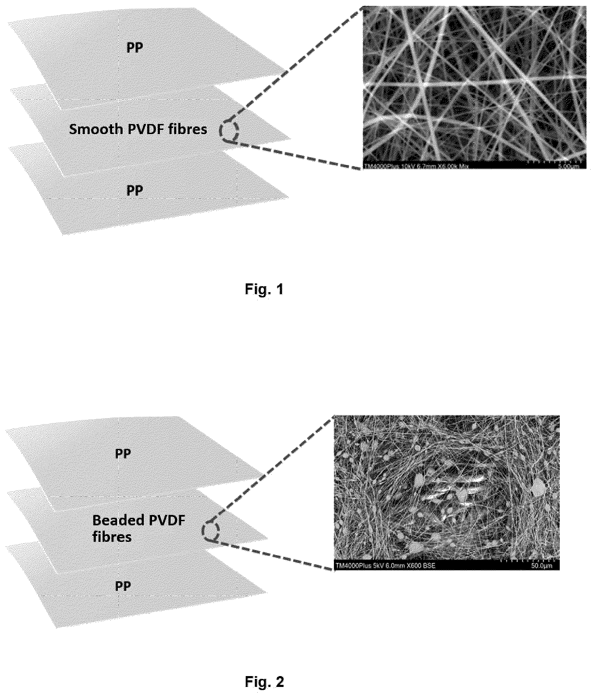

The central layer of electrospun ultrafine fibres was made of polyvinylidene fluoride with a molecular weight of 300 kDalton. To do this, a solution of PVDF at 15% by weight (wt.%) in a DMF/Acetone mixture (50:50 wt.) was used. Once dissolved, the fibre sheet was then manufactured using the electrospinning technique. To do this, an emitter voltage of 18kV and a linear multi-emitter injector voltage were used. These ultrafine fibres were deposited on a rotating collector at a speed of 200 revolutions per minute (rpm) on a 30 g/m² polypropylene (PP) substrate and at a distance of 20 cm. Said manufacture was carried out at a temperature of 30 °C and a relative humidity of 30%. This layer has a surface density of 1 g/m². After production, a 30 g/m² PP layer was placed on the PVDF deposition and calendered at 80 °C so that the final material ends up like the multilayer filter described in Figure 1.

| | Material | Grammage (g/m²) |
|---|---|---|
| Outer Layer | Non-woven PP spunbond | 30 |
| Intermediate Layer | Electrospun PVDF with smooth fibres | 1 |
| Inner Layer | Non-woven PP spunbond | 30 |

The PVDF layer generated by the electrospinning technique was observed with a scanning electron microscope (SEM), resulting in a fibre microstructure with a constant diameter of between 220 and 280 nm, as can be seen in Figure 1. When this material is subsequently subjected to a washing cycle with stirring in hot water at 60 °C and detergent and then dried, the consistency and morphology of the intermediate layer measured by SEM is not affected.

Assays of resistance to penetration with paraffin aerosol according to standard 149:2001+A1:2009 (point 8.11) gave a value of 0.9%; therefore, this filter would be classified as FFP3 type (out of every 100 aerosol particles, 1 or less than 1 passes).

### Example 2: FFP1 tri-layer structure system with electrospun PVDF with beaded ultrafine fibre structure

The central layer was made of polyvinylidene fluoride with a molecular weight of 500 kDalton. To do this, a solution of PVDF at 10% by weight (wt.%) in a DMF/Acetone mixture (50:50 wt.) was used. Once dissolved, the fibre sheet was then manufactured using the electrospinning technique. To do this, an emitter voltage of 19kV and a collector voltage of -7kV were used. A flow rate of 10 ml/h through a linear multi-emitter injector was also used. The fibres were deposited on a rotating collector at 200 rpm covered by a 30 g/m² non-woven PP substrate and at a distance of 20 cm. Said manufacture was carried out at a temperature of 30 °C and a relative humidity of 30%. This layer has a surface density of 3 g/m². After production, a 30 g/m² PP layer was placed on the PVDF deposition and calendered at 80 °C so that the final material ends up like the multilayer filter illustrated in Figure 2.

| | Material | Grammage (g/m²) |
|---|---|---|
| Outer Layer | Non-woven PP spunbond | 30 |
| Intermediate Layer | Electrospun PVDF with beaded fibres | 3 |
| Inner Layer | Non-woven PP spunbond | 30 |

The PVDF layer generated by electrospinning was observed with a scanning electron microscope (SEM), resulting in a fibre structure of around 200 nanometres with micrometre-sized beaded structures, which correspond to areas of the fibres where their size increases considerably forming a type of particle, thus being called beaded fibres. This beaded morphology provides advantages in the breathability of the fabric since the beads help to optimise the packing density of the fibre and the presence thereof increases the distance between the fibres to reduce the pressure drop on the filters.

Assays of resistance to penetration with paraffin oil according to standard 149:2001+A1:2009 (point 8.11) gave a value of 17.8%; therefore, this filter would be classified as FFP1 type (out of every 100 aerosol particles, 20 or less than 20 pass).

### Example 3: FFP2 tri-layer structure system with smooth ultrafine electrospun PAN and Zinc Oxide fibres

The central layer was made of polyacrylonitrile (PAN). To do this, a solution of PAN at 11% by weight (wt.%) with dimethylformamide (DMF) and zinc oxide (ZnO) nanoparticles in a percentage of 2 by weight (wt.%) was used to generate antimicrobial properties. Once dissolved, the fibre sheet was then manufactured using the electrospinning technique. To do this, an emitter voltage of 30kV and a collector voltage of -10kV were used, and a flow rate of 5 ml/h through a linear multi-emitter injector was also used. The fibres were deposited on a rotating collector at a speed of 200 rpm covered by a 30 g/m² non-woven PP substrate and at a distance of 20 cm. Said manufacture was carried out at a temperature of 30 °C and a relative humidity of 30%. This layer has a surface density of 0.5 g/m². After production, a 30 g/m² non-woven PP layer was placed on the PAN deposition and calendered at 80 °C so that the final material ends up similar to the multilayer filter described in Figure 1.

| | Material | Grammage (g/m²) |
|---|---|---|
| Outer Layer | Non-woven PP spunbond | 30 |
| Intermediate Layer | Electrospun PAN with smooth fibres | 0.5 |
| Inner Layer | Non-woven PP spunbond | 30 |

Likewise, the antimicrobial properties of this structure were evaluated using a modification of the Japanese Industrial Standard JIS Z 2801 (ISO 22196:2007) against the strains of *Staphylococcus aureus* (*S*. *aureus*) CECT240 (ATCC 6538p) and *Escherichia coli* (*E. coli*) CECT434 (ATCC 25922). The filters were analysed in terms of the capacity to inhibit the growth of these populations in the material and it was observed, as illustrated in Table 1, that the filters showed strong growth inhibition of both strains (R≥3) with a reduction of 3 recorded units with respect to the control (filters without ZnO) on the first day of measuring it. These results indicate that these filters efficiently inhibit this type of strain, since an R <0.5 would indicate that the inhibition of the material towards bacteria is not significant, while an R ≥ 1 and <3 would indicate that it is slightly significant. An R ≥ 3 would indicate that it is clearly significant, which means that the inhibition of the growth of microorganisms is effective and constant over time.

**Table 1. Reduction of S. aureus and E. coli on filters with antimicrobial capacity after 24 hours.**

| **Microorganism** | **Days** | **Control (PAN filter) Log (CFU/ml)** | **PAN + 2% ZnO filter Log (CFU/ml)** | **R** |
|---|---|---|---|---|
| *S*. *Aureus* | 1 | 6.91 ± 0.06 | 3.00 ± 0.05 | 3.91 |
| *E.coli* | 1 | 6.91 ± 0.06 | 3.78 ± 0.08 | 3.13 |

Assays of resistance to penetration with paraffin oil according to standard 149:2001+A1:2009 (point 8.11) gave a value of 5%; therefore, this filter would be classified as FFP2 type (of every 100 aerosol particles, 6 or less than 6 pass).

### Example 4: Bactericidal properties as a function of the contact time of an intermediate layer of smooth ultrafine electrospun PAN and Zinc Oxide fibres

The central nanofibre layer was made of polyacrylonitrile (PAN). To do this, a solution of PAN at 11% by weight (wt.%) with dimethylformamide (DMF) and zinc oxide (ZnO) nanoparticles with a percentage of 3 by weight (wt.%) was used to generate antimicrobial properties. Once dissolved, the fibre sheet was then manufactured using the electrospinning technique. To do this, an emitter voltage of 30kV and a collector voltage of -10kV were used, and a flow rate of 5 ml/h through a linear multi-emitter injector was also used. The fibres were deposited on a rotating collector at a speed of 200 rpm covered by a black conductive non-porous polyethylene substrate. The nanofibre layer had a surface density of 0.4 g/m².

The antimicrobial properties of this structure were evaluated using a modification of the Japanese Industrial Standard JIS Z 2801 (ISO 22196:2007) against the strains of *Staphylococcus aureus* (*S. aureus*) CECT240 (ATCC 6538p) and *Escherichia coli* (*E. coli*) CECT434 (ATCC 25922) over a period of up to 8 hours. The filters were analysed in terms of the capacity to inhibit the growth of these populations in the material and it was observed, as illustrated in Table 2, that the filters showed strong growth inhibition of both strains (R≥3) with a reduction of 3 recorded units with respect to the control (filters without ZnO) at 3 hours of contact. These results indicate that these filters efficiently inhibit this type of strain, since an R <0.5 would indicate that the inhibition of the material towards bacteria is not significant, while an R ≥ 1 and <3 would indicate that it is slightly significant. An R ≥ 3 would indicate that it is clearly significant, which means that the inhibition of the growth of microorganisms is effective and constant over time.

**Table 2. Reduction of S. aureus and E. coli on filters with antimicrobial capacity after 1, 3, 6 and 8 hours of contact.**

| | **Time (h)** | **Control Log (CFU/ml)** | **Nanofibres Log (CFU/ml)** | **R** |
|---|---|---|---|---|
| ***S*. *aureus*** | 1 | 6.01 ± 0.11 | 3.76 ± 0.21 | 2.25 (99%) |
| | 3 | 6.86 ± 0.17 | 3.68 ± 0.15 | 3.18 (99.9%) |
| | 6 | 7.18 ± 0.19 | 3.10 ± 0.18 | 4.08 (99.99%) |
| | 8 | 7.86 ± 0.13 | 2.99 ± 0.17 | 4.87 (99.999%) |
| ***E. coli*** | 1 | 5.98 ± 0.09 | 3.96 ± 0.11 | 2.02 (99%) |
| | 3 | 6.36 ± 0.10 | 3.29 ± 0.13 | 3.07 (99.9%) |
| | 6 | 7.01 ± 0.14 | 3.32 ± 0.10 | 3.69 (99.99%) |
| | 8 | 7.88 ± 0.11 | 3.28 ± 0.15 | 4.60 (99.999%) |

### Example 5: Bactericidal properties as a function of the time of a surgical mask with smooth ultrafine electrospun PVDF and Zinc Oxide fibres.

The central layer was made of polyvinylidene fluoride. To do this, a solution of PVDF at 13% by weight (wt.%) in a DMF/Acetone mixture (50:50 wt.) and with 3 percent by weight of ZnO (wt.%) with respect to the polymer was used. This solution was sonicated for 3 minutes before being electrospun, and then the fibre sheet was manufactured. To do this, an emitter voltage of 30 kV and a collector voltage of -10kV were used, and a flow rate of 5 ml/h through a linear multi-emitter injector was also used. These ultrafine fibres were deposited on a roll-to-roll system in LE-500-Fluidnatek equipment from Bioinicia SL on a 17 g/m² polypropylene (PP) spunbond substrate and at a distance of 20 cm. Said manufacture was carried out at a temperature of 30 °C and a relative humidity of 30%. This layer had a surface density of 0.3 g/m². It was then laminated to another 17 g/m² PP spunbond layer in a laminator and sealed on the edges with ultrasound. This roll was used to make a surgical mask sealed by stitching and adding two more 30 g/m² PP spunbond layers to each side.

| | Material | Grammage (g/m²) |
|---|---|---|
| Double Outer Layer | Non-woven PP spunbond | 30 and 17 |
| Intermediate Layer | Electrospun PVDF with smooth fibres | 0.3 |
| Double Inner Layer | Non-woven PP spunbond | 17 and 30 |

The bactericidal performance of this mask configuration was determined according to the guidelines of the macrodilution protocol, which is described in Methods for Dilution Antimicrobial Susceptibility Tests for Bacteria That Grow Aerobically; Approved Standard-Tenth. Edition (M07-A10) by the Clinical and Laboratory Standards Institute (CLSI)). The antibacterial properties of this structure were evaluated against strains of *Staphylococcus aureus* (*S. aureus*) CECT240 (ATCC 6538p) and *Escherichia coli* (*E. coli*) CECT434 (ATCC 25922) over 8 hours and the results are shown in Table 3.

In this case, it was observed that the mask showed strong growth inhibition of both strains with a reduction of 3 recorded units with respect to the control (same mask, but without ZnO) after 8 hours of contact, showing that the antimicrobial activity after 1 hour already indicated that there was a slightly significant inhibition of the mask against both strains. An R <0.5 would indicate that the inhibition of the material towards bacteria is not significant, while an R ≥ 1 and <3 would indicate that it is slightly significant. An R ≥ 3 would indicate that it is clearly significant, which means that the inhibition of the growth of microorganisms is effective and constant over time (See Table 3).

**Table 3. Reduction of S. aureus and E. coli in a surgical mask with bactericidal capacity after 1, 3, 6 and 8 hours of contact.**

| | **Time (h)** | **Control Log (CFU/ml)** | **Mask Log (CFU/ml)** | **R** |
|---|---|---|---|---|
| ***S*. *aureus*** | 1 | 6.06 ± 0.21 | 4.53 ± 0.25 | 1.53 (95%) |
| | 3 | 6.91 ± 0.20 | 4.33 ± 0.18 | 2.58 (99.6%) |
| | 6 | 7.23 ± 0.16 | 4.22 ± 0.17 | 3.01 (99.9%) |
| | 8 | 7.91 ± 0.19 | 4.10 ± 0.20 | 3.81 (99.99%) |
| ***E. coli*** | 1 | 6.03 ± 0.18 | 4.61 ± 0.15 | 1.42 (95%) |
| | 3 | 6.42 ± 0.21 | 4.19 ± 0.20 | 2.23 (99%) |
| | 6 | 7.11 ± 0.17 | 4.15 ± 0.19 | 2.96 (99.9%) |
| | 8 | 7.89 ± 0.15 | 4.12 ± 0.13 | 3.77 (99.99%) |

### Example 6: FFP2 tri-layer structure system with electrospun PHBV with smooth ultrafine fibre structure

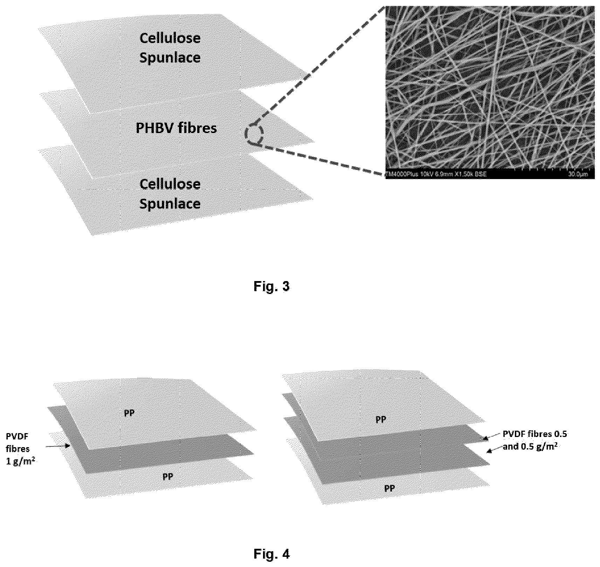

The central layer was made of poly(3-hydroxybutyrate-co-3-hydroxyvalerate) (PHBV) supplied by Ocenic Resins SL, Valencia. To do this, a solution of PHBV at 2% by weight (wt.%) in trifluoroethanol (TFE) was used. Once dissolved, it was manufactured with and without the addition of LiBr (0.2 wt%) and the fibre sheet was then manufactured using the electrospinning technique. To do this, an emitter voltage of 18kV and a collector voltage of -8kV were used, and a flow rate of 20 ml/h through a linear multi-emitter injector was also used. The fibres were deposited on a rotating collector at a speed of 200 rpm covered by a 30 g/m² biodegradable non-woven cellulose spunlace substrate and at a distance of 20 cm. Said manufacture was carried out at a temperature of 30 °C and a relative humidity of 30%. This layer has a surface density of 1 g/m². After production, a 30 g/m² biodegradable non-woven cellulose spunlace layer was placed on the PHBV deposition and calendered at 80 °C so that the final material ends up like the multilayer filter described in Figure 3.

| | Material | Grammage (g/m²) |
|---|---|---|
| Top Layer | Cellulose spunlace | 30 |
| Intermediate Layer | Electrospun PHBV | 1 |
| Lower Layer | Cellulose spunlace | 30 |

The PHBV layer generated by electrospinning was observed with a scanning electron microscope, resulting in a fibre microstructure with a constant diameter of between approximately 200 and 300 nm, as can be seen in Figure 4.

Biodisintegration assays were carried out according to ISO 20200 "Plastics - Determination of the degree of disintegration of plastic materials under simulated composting conditions in a laboratory-scale test". The PHBV filter could be considered fully compostable according to ISO 20200 since the disintegration process of the PHBV nanofibre layer reached total disintegration after 20 days of assays. This short degradation time is probably related to the low thickness of the nanofibre layer of the filter, necessary for good breathing. The multilayer system reached complete disintegration in 80 days.

Assays of resistance to penetration with paraffin oil according to standard 149:2001+A1:2009 (point 8.11) gave a value of 5.5%; therefore, this filter would be classified as FFP2 type (of every 100 aerosol particles, 6 or less than 6 pass).

### Example 7: FFP3 multilayer structure system with sequentially electrospun PVDF arranged as a symmetrical sandwich

The central layer was made of polyvinylidene fluoride (PVDF, molecular weight 300 kDalton) at 13% by weight of DMF/Acetone (50:50 wt.). Once the solution was dissolved, the fibre sheet was then manufactured using the electrospinning technique. To do this, an emitter voltage of 25kV, as well as a collector voltage of -10kV, was used. A flow rate of 10 ml/h through a linear multi-emitter injector was also used. The fibres were deposited on a rotating collector (200 rpm) covered by a 30 g/m² non-woven PP substrate and at a distance of 20 cm. Said manufacture was carried out at a temperature of 30 °C and a relative humidity of 30%. This layer has a surface density of 1 g/m².

This same layer was prepared in duplicate under the same conditions, but at 0.5 g/m² on a 30 g/m² non-woven PP layer and it was folded like a symmetrical sandwich, so that the structure would be as shown in Figure 4. This structure improves filtration performance because it fixes the fibres on the substrate and the entire filter is adhered by interaction between the nanofibres.

The PVDF layers generated by electrospinning were observed with a scanning electron microscope where a constant diameter of between approximately 200 and 300 nm was obtained. When this material was subsequently subjected to a washing cycle with stirring in hot water at 60 °C and detergent and then dried, the consistency and morphology of the intermediate layer measured by SEM is not affected.

Assays of resistance to penetration with paraffin oil according to standard 149:2001+A1:2009 (point 8.11) gave a value for the 1 g/m² monolayer fibre structure of 2.3% (FFP2 type), while the symmetric sandwich double layer structure gave 0.9%. Therefore, the latter filter would be classified as FFP3 type (of every 100 aerosol particles, 1 or less than 1 passes).

Resistance to inhalation was measured according to EN149: 2001+A1:2009 (point 8.9) over an area of approximately 53 cm² on Sheffield test head equipment with constant breathing and a digital flow meter. Respiration results for the monolayer were 0.7 millibars for an air flow of 30 l/min; and results for the double layer structure were 0.8 millibars, within the limits of the FFP3 certification. Inhalation assays carried out at 85 l/min, as recommended by the N95 certification, gave values of 3.3 for the double-layer structure, within the limits of N95.

### Example 8: FFP3 tri-layer structure system with several layers electrospun by co-deposition

The central layer was made of polyvinylidene fluoride (PVDF, molecular weight 300 kDalton) and polyacrylonitrile (PAN) to obtain a filter with different fibre diameters. To do this, a solution of PVDF at 13% by weight (wt.%) in DMF/Acetone (50:50 wt.) and a solution of PAN at 11% by weight (wt.%) in DMF were used. Once both solutions were dissolved, the fibre sheet was then manufactured using the electrospinning by co-deposition technique, wherein both types of fibres are simultaneously electrospun by two linear multi-emitter injectors. To do this, an emitter voltage of 18kV and 25 kV was used for the solution of PVDF and PAN, respectively, as well as a collector voltage of -30kV. A flow rate of 13.8 ml/h for PVDF and 3 ml/h for PAN through 2 linear multi-emitter injectors placed in parallel was also used. The fibres were deposited on a rotating collector (200 rpm) covered by a 30 g/m² non-woven PP substrate and at a distance of 20 cm. Said manufacture was carried out at a temperature of 30 °C and a relative humidity of 30%. This layer has a surface density of 1.2 g/m².

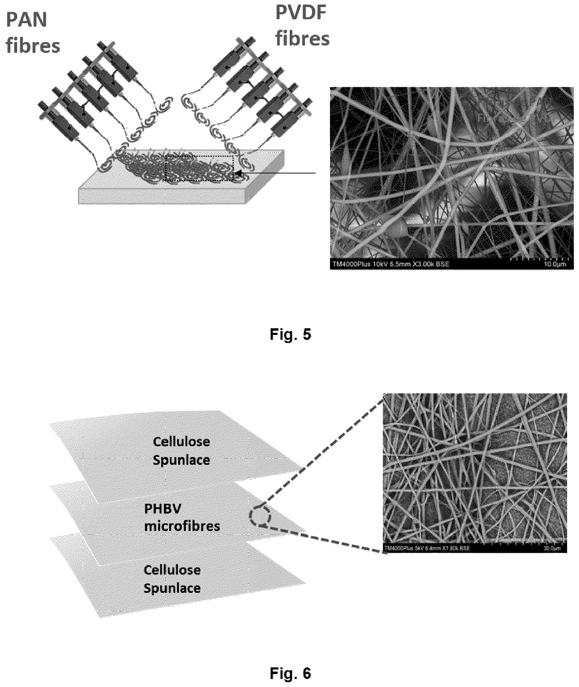

After production, a 30 g/m² non-woven PP layer was placed on the PAN deposition and calendered at 80 °C so that the final material ends up like the multilayer filter illustrated in Figure 5.

| | Material | Grammage (g/m²) |
|---|---|---|
| Top Layer | Non-woven PP spunbond | 30 |
| Intermediate Layer | Co-electrospun PVDF and PAN | 1.2 (1 PVDF + 0.2 PAN) |
| Lower Layer | Non-woven PP spunbond | 30 |

The PVDF and PAN layer generated by electrospinning was observed with a scanning electron microscope where a constant diameter of between approximately 150 and 250 nm for the PAN fibres and a diameter of between 300-500 nm for the PVDF fibres were obtained.

Assays of resistance to penetration with paraffin oil according to standard 149:2001+A1:2009 (point 8.11) gave a value for the 1.2 g/m² structure of 0.6%. Therefore, the latter filter would be classified as FFP3 type (of every 100 aerosol particles, 1 or less than 1 passes).

### Example 9: Electrospun tri-layer structure system with micrometric fibres

The central layer was made of poly(3-hydroxybutyrate-co-3-hydroxyvalerate) (PHBV) supplied by Ocenic Resins SL, Valencia. To do this, a solution of PHBV at 6% by weight in trifluoroethanol (TFE) was used. Once dissolved, the fibre sheet was then manufactured using the electrospinning technique. To do this, an emitter voltage of 15kV and a collector voltage of -8kV were used, a flow rate of 20 ml/h through a multi-emitter injector was also used. The fibres were deposited on a rotating collector at a speed of 200 rpm covered by a 30 g/m² biodegradable non-woven cellulose spunlace substrate and at a distance of 20 cm. Said manufacture was carried out at a temperature of 30 °C and a relative humidity of 30%. This layer has a surface density of 0.5 g/m². After production, a 30 g/m² non-woven cellulose spunlace layer was placed on the PHBV deposition.

The PHBV layer generated by electrospinning was observed with a scanning electron microscope, resulting in a fibre microstructure with a constant diameter of 900-1200 nm, as can be seen in Figure 6.

Assays of resistance to penetration with paraffin oil according to standard 149:2001+A1:2009 (point 8.11) gave a value for the 0.5 g/m² monolayer structure of 87%, corroborating the need to obtain ultrafine fibres for this particular application.

### Example 10: Preparation of a viricidal intermediate layer of smooth ultrafine electrospun PVDF and Zinc Oxide fibres

A solution of PVDF at 13% by weight (wt.%) in a DMF/Acetone mixture (50:50 wt.) and with amounts of ZnO particles of 3, 20 and 30 by weight (wt.%) with respect to the polymer. These solutions were sonicated for 3 minutes before being electrospun, and then the fibres were deposited. To do this, an emitter voltage of 30 kV and a collector voltage of -10kV were used, and a flow rate of 5 ml/h through a linear multi-emitter injector was also used. These ultrafine fibres were deposited on a rotating collector at a speed of 200 revolutions per minute (rpm) on a black conductive non-porous polyethylene substrate. Said manufacture was carried out at a temperature of 30 °C and a relative humidity of 30%. This nanofibre layer had a surface density of 0.3 g/m².

The viricidal properties of the nanofibres produced were studied on this system. To do this, the standard for determining antiviral activity in textiles (ISO 18184:2019) against a feline coronavirus strain (*Feline Coronavirus, strain Munich*) was used. These assays were performed at the certified MSL Solutions Providers facility, Bury, GB. As can be observed in Table 4, the nanofibre layer without the antimicrobial agent showed a certain antiviral nature, probably due to the nanometric topography of the material. However, the addition of the viricidal agent showed very strong inhibition of up to 97.13% for the highest content. The viricidal effect did not significantly increase with the increase in ZnO content, probably because higher contents lead to the agglomeration of antimicrobial particles.

**Table 4. Percentage of growth inhibition against feline coronavirus after 2 hours of contact.**

| **Sample** | **Reduction** | **Percentage of inhibition at 2 hours of contact** |
|---|---|---|
| Control (PVDF fibres without ZnO) | 0.58 | 73.90% |
| PVDF fibres + ZnO at 3 wt.% | 1.44 | 96.41% |
| PVDF fibres + ZnO at 20 wt.% | 1.39 | 95.92% |
| PVDF fibres + ZnO at 30 wt.% | 1.54 | 97.13% |

## Claims

1. A multilayer filter **characterised in that** it comprises at least:
i) An inner layer (a) **characterised in that** it is composed of polymeric filter materials and has a surface density of at least 0.01 g/m², more preferably between 5 and 3000 g/m², even more preferably between 20 and 300 g/m²;
ii) An intermediate layer (b) **characterised in that** it is composed of polymeric fibres, optionally containing antimicrobial substances, and has a surface density of at least 0.01 g/m², more preferably between 0.1 and 10 g/m², and even more preferably between 0.2 and 3 g/m²;
iii) An outer layer (c) **characterised in that** it is composed of polymeric filter materials and has a surface density of at least 0.01 g/m², more preferably between 5 and 3000 g/m², and even more preferably between 20 and 300 g/m².

2. The filter according to the preceding claim, wherein the inner layer (a) and the outer layer (c) are made of woven or non-woven polymeric filter materials, with or without functional additives.

3. The filter according to the preceding claim, wherein the inner layer (a) and the outer layer (c) are made of non-woven polymeric filter materials.

4. The filter according to any of the preceding claims, wherein the polymeric material that makes up the inner layer (a) and the outer layer (c) are independently selected from polypropylene, polyamide, polyester, natural fibres, cotton and cellulose, or any of the combinations thereof.

5. The filter according to any of the preceding claims, wherein the fibres forming the intermediate layer (b) are selected from polyvinylidene chloride, polyacrylonitrile and polyhydroxyalkanoates, or any of the combinations thereof.

6. The filter according to the preceding claim, wherein the fibres forming the intermediate layer (b) are made of polyvinylidene chloride or polyhydroxyalkanoates.

7. The filter according to any of the preceding claims, wherein the fibres forming the intermediate layer (b) have a molecular weight less than 200 kDalton.

8. The filter according to any of the preceding claims, wherein the intermediate layer (b) contains an antimicrobial substance selected from zinc oxide, silver, silver nitrate, copper, copper oxide, graphene, carbon microtubes, carbon nanotubes, titanium oxide, titanium dioxide, natural extracts, essential oils, chitin, chitosan, aluminium oxide, silicon dioxide, cyclodextrins, antibiotics, tetracycline, iodine, triclosan, chlorhexidine, acyclovir and cyclofloxacin, or combinations thereof.

9. The filter according to the preceding claim, wherein the antimicrobial substance is zinc oxide or zinc oxide nanoparticles.

10. The filter according to any of claims 1 to 7, wherein the intermediate layer (b) does not contain an antimicrobial substance.

11. The filter according to any of the preceding claims, wherein the fibres of the intermediate layer (b) are fibres of a smooth or beaded morphology.

12. The filter according to any of the preceding claims, wherein the intermediate layer (b) comprises an additional layer (b'), **characterised in that** it is composed of the same polymeric fibres as those of the first intermediate layer (b) on which it is deposited.

13. The filter according to any of claims 1 to 11, wherein the intermediate layer (b) comprises an additional layer (b'), **characterised in that** it is composed of polymeric fibres different from those of the first intermediate layer (b) on which it is deposited.

14. The filter according to any of claims 12 to 13, wherein the morphology of the polymeric fibres that make up the intermediate layers (b) and (b') is the same.

15. The filter according to any of claims 12 to 13, wherein the morphology of the polymeric fibres that make up the intermediate layers (b) and (b') is different.

16. The filter according to claim 14, wherein the surface density of the intermediate layers (b) and (b') is the same.

17. The filter according to claim 14, wherein the surface density of the intermediate layers (b) and (b') is different.

18. The filter according to claim 15, wherein the surface density of the intermediate layers (b) and (b') is the same.

19. The filter according to claim 15, wherein the surface density of the intermediate layers (b) and (b') is different.

20. The filter according to any of claims 13 to 20, wherein the fibres of the additional intermediate layer (b') have a morphology with fibres with a diameter greater than 500 nm.

21. The filter according to any of the preceding claims, wherein the dispersion of the surface density of the intermediate layer (b) is less than 10%.

22. The filter according to any of the preceding claims, wherein the polymers that make up the filter layers are preferably compostable and/or biodegradable in the environment.

23. The filter according to any of the preceding claims, wherein said filter can be stacked in any possible configuration on themselves or on other commercial multilayer or monolayer filters.

24. A method for obtaining a multilayer filter according to any of claims 1 to 23 comprising the following steps:
i) Depositing the intermediate layer (b) on the inner layer (a);
ii) Optionally, depositing one or more additional intermediate layers (b') on the intermediate layer (b);
iii) Laminating the outer layer (c) with the previous layers.

25. A method for obtaining a multilayer filter according to any of claims 1 to 24 comprising the following steps:
i) Depositing the intermediate layer (b) on the inner layer (a);
ii) Depositing the additional intermediate layer (b') on the inner face of the outer layer (c);
iii) Laminating the previous layers so that layers (b) and (b') are in contact.

26. The method according to any of claims 24 and 25, wherein the layers (a), (b), (b') and (c) are laminated by simply joining the layers together, without any type of adhesion method.

27. The method according to any of claims 24 and 25, wherein layers (a), (b), (b') and (c) are partially or totally laminated along the surface thereof, adding protective layers below the inner layer (a) and/or above the outer layer (c) or not, by means of methods that are selected from calendering with pressure, calendering without pressure, applying adhesives, with melting points by ultrasound, stitching and heat-sealing.

28. The filter according to any of claims 1 to 23 for use in the manufacture of medical ventilators and washable or non-washable protective masks.

29. The filter according to the preceding claim, wherein the masks are surgical or hygienic masks.

30. The filter according to any of claims 1 to 23 for use in the manufacture of personal protective equipment.

31. The filter according to any of claims 1 to 23 for use in the manufacture of generic industrial air or liquid filtration equipment.

32. A mask containing the filter according to any of claims 1 to 23, wherein the mask protects against microorganisms.

33. The mask according to the preceding claim, wherein the microorganism is a virus that is selected from Ebola virus, herpes virus, influenza virus, human respiratory syncytial virus, chickenpox, SARS-CoV and the derivatives thereof, as well as SARS-CoV-2 which causes COVID-19.

34. Generic industrial air or liquid filtration equipment containing the filter according to any of claims 1 to 23, wherein the filter removes microorganisms.

35. The filtration equipment according to the preceding claim, wherein the microorganism is a virus that is selected from Ebola virus, herpes virus, influenza virus, human respiratory syncytial virus, chickenpox, SARS-CoV and the derivatives thereof, as well as SARS-CoV-2 which causes COVID-19.
